# EUROPEAN PATENT APPLICATION

(11) **EP 4 652 950 A1**
(43) Date of publication of application: **26.11.2025**
(21) Application number: 25177644.9
(22) Date of filing: 20.05.2025
(51) Int. Cl.: A61B 34/00, A61B 34/20, A61B 17/00, A61B 34/10

(54) **SYSTEM FOR REDUCING LATENCY DURING TRACKING A HANDHELD SURGICAL INSTRUMENT USING MESH TO MESH COLLISIONS AND A MODIFIED END EFFECTOR MODEL**

(30) Priority: 21.05.2024 US 202463650055 P
(71) Applicant: Stryker European Operations Limited, Carrigtwohill, Co. Cork T45 HX08 (IE)
(72) Inventor: Becher, Jochen, 79263 Simonswald (DE); Hertel, Julius Maximilian, 79114 Freiburg (DE); Schwalbe, Lars Marius, 79283 Bollschweil (DE)
(74) Representative: Schott, Jakob Valentin

(57) **Abstract**

A surgical navigation system is provided, which includes a handheld surgical instrument configured to receive an end effector, a localizer configured to determine a pose of the handheld surgical instrument, an alert module for providing notifications to a user, and a control system in communication with the localizer and the alert module. The control system configured to receive a selection of the end effector, associate an end effector model with the handheld surgical instrument based on the selection of the end effector, receive a selection of a security margin, modify a spatial dimension of the end effector model based on the selected security margin, determine a pose of the modified end effector model, determine a pose of a patient boundary associated with a target anatomical feature, and control the alert module based on the pose of the patient boundary and the pose of the modified model of the end effector.

## Description

### BACKGROUND

Surgical navigation systems assist in providing precision and accuracy during surgical procedures. Surgical navigation systems typically include means for tracking the position and location of surgical instruments relative to a patient being operated on. Tracking the position and location of surgical instruments relative to patient boundaries, such as those surrounding a target anatomical feature, may require creating a model of the surgical instrument. However, tracking the surgical instrument and its relation to patient boundaries may be computationally expensive to do in real time.

### SUMMARY

This Summary introduces a selection of concepts in a simplified form that are further described below in the Detailed Description below. This Summary is not intended to limit the scope of the claimed subject matter nor identify key features or essential features of the claimed subject matter.

According to a first aspect, a surgical navigation system is provided. The surgical navigation system includes a handheld surgical instrument configured to receive an end effector, a localizer configured to determine a pose of the handheld surgical instrument, an alert module for providing notifications to a user, and a control system in communication with the localizer and the alert module. The control system configured to receive a selection of the end effector, associate an end effector model with the handheld surgical instrument based on the selection of the end effector, receive a selection of a security margin, modify a spatial dimension of the end effector model based on the selected security margin, determine a pose of the modified end effector model, determine a pose of a patient boundary associated with a target anatomical feature, and control the alert module based on the pose of the patient boundary and the pose of the modified model of the end effector.

According to a second aspect, a method of tracking an end effector of a handheld surgical instrument relative to a target anatomical feature is provided. The method comprising receiving a selection of the end effector, associating an end effector model with the handheld surgical instrument based on the election of the end effector, receiving a selection of a security margin, modifying a spatial dimension of the end effector model based on the selected security margin, determining a pose of the modified end effector model, determining a pose of a patient boundary associated with the target anatomical feature, and controlling an alert module based on the pose of the patient boundary and the pose of the modified model of the end effector.

According to a third aspect, a method of tracking an end effector of a handheld surgical instrument relative to a target anatomical feature is provided. The method comprising receiving a selection of the end effector, associating an end effector model with the handheld surgical instrument based on the selection of the end effector, receiving a selection of a security margin, modifying a spatial dimension of the end effector model based on the selected security margin, determining a pose of the modified end effector model, determining a pose of a patient boundary associated with the target anatomical feature, and controlling the handheld surgical instrument based on the pose of the patient boundary and the pose of the modified model of the end effector.

According to a fourth aspect, a surgical navigation system is provided. The surgical navigation system comprises a handheld surgical instrument configured to receive an end effector, a localizer configured to determine a pose of the handheld surgical instrument, and a control system in communication with the localizer. The control system is configured to receive a selection of the end effector, associate an end effector model with the handheld surgical instrument based on the selection of the end effector, receive a selection of a security margin, modify a spatial dimension of the end effector model based on the selected security margin, determine a pose of the modified end effector model, determine a pose of a patient boundary associated with a target anatomical feature, and control the handheld surgical instrument based on the pose of the patient boundary and the pose of the modified model of the end effector.

According to a fifth aspect, a surgical navigation system is provided. The surgical navigation system comprises a control system in communication with a localizer and an alert module. The control system configured to receive a selection of the end effector, associate an end effector model with the handheld surgical instrument based on the selection of the end effector, receive a selection of a security margin, modify a spatial dimension of the end effector model based on the selected security margin, determine a pose of the modified end effector model, determine a pose of a patient boundary associated with a target anatomical feature, and control the alert module based on the pose of the patient boundary and the pose of the modified model of the end effector.

According to a sixth aspect, a surgical navigation system is provided. The surgical navigation system comprises a control system in communication with a localizer. The control system configured to receive a selection of the end effector, associate an end effector model with a handheld surgical instrument based on the selection of the end effector, receive a selection of a security margin, modify a spatial dimension of the end effector model based on the selected security margin, determine a pose of the modified end effector model, determine a pose of a patient boundary associated with a target anatomical feature, and control the handheld surgical instrument based on the pose of the patient boundary and the pose of the modified model of the end effector.

Any of the above aspects can be combined in part or in whole with any other aspect. Any of the above aspects, whether combined in part or in whole, can be further combined with any of the following implementations, in full or in part.

In one implementation, the surgical navigation system may further include a console in electrical communication with the handheld surgical instrument. In one implementation, the surgical navigation system may further include a patient tracker coupled to a patient, the localizer is configured to track the patient tracker, and the control system is configured to associate the patient boundary of the target anatomical feature with the patient by associating the pose of the patient boundary with a pose of the patient tracker. In one implementation, the surgical navigation system may further include the control system configured to receive the selection of the security margin based on the selection of the end effector. In one implementation, the surgical navigation system may further include the control system configured to receive the selection of the security margin based on a selection of a type of surgical procedure.

In one implementation, the surgical navigation system may further include the security margin is based on at least one of the following: a current stage of a surgical procedure, and/or the pose of the patient boundary associated with the target anatomical feature. In one implementation, the surgical navigation system may further include the security margin based on a user input. In one implementation, the surgical navigation system may further include the user input including a type of end effector.

In one implementation, the surgical navigation system may further include the end effector model is a polygon mesh. In one implementation, the surgical navigation system may further include the end effector model including a resolution and the control system is configured to select the resolution based on a current stage of a surgical procedure, a type of end effector, and/or the pose of the patient boundary associated with the target anatomical feature. In one implementation, the surgical navigation system may further wherein the patient boundary is a polygon mesh.

In one implementation, the surgical navigation system may further include wherein the control system is further configured to control the alert module based on a collision between the modified end effector model and the patient boundary. In one implementation, the surgical navigation system may further include wherein the alert module is selected from a group including: a display device; a haptic feedback device; and/or a speaker.

In one implementation, the surgical navigation system may further include wherein the alert module is the display device and the control system is further configured to control the display device to display a notification in response to a collision between the modified end effector model and the patient boundary. In one implementation, the surgical navigation system may further include wherein the alert module is the haptic feedback device and the control system is further configured to control the haptic feedback device to provide a tactile feedback in response to a collision between the modified end effector model and the patient boundary. In one implementation, the surgical navigation system may further include wherein the alert module is the speaker and the control system is further configured to control the speaker to output an audible alert in response to a collision between the modified end effector model and the patient boundary.

In one implementation, the surgical navigation system may further include wherein the selection of the end effector is realized as a first selection of the end effector and a second selection of the end effector and the control system is further configured to: disassociate the first end effector model with the handheld surgical instrument, relinquish control of the alert module based on the pose of the patient boundary and the pose of the modified model of the first end effector, receive the second selection of the end effector, receive the selection of the second security margin, modify a spatial dimension of the second end effector model based on the selected second security margin, determine a pose of the modified second end effector model, determine the pose of the patient boundary associated with the target anatomical feature, and control the alert module based on the pose of the patient boundary and the pose of the modified model of the second end effector.

In one implementation, the surgical navigation system may further include wherein the end effector includes a non-spherical bur. In one implementation, the surgical navigation system may further include wherein the handheld surgical instrument includes a drive motor. In one implementation, the surgical navigation system may further include wherein the control system, in response to a collision between the modified end effector model and the patient boundary, is further configured to reduce an operating speed of the drive motor of the handheld surgical instrument. In one implementation, the surgical navigation system may further include wherein the operating speed is reduced to a non-zero speed. In one implementation, the surgical navigation system may further include wherein the operating speed is reduced to a speed of zero.

In one implementation, the surgical navigation system may further include wherein the selection of the end effector is realized as a first selection of the end effector and a second selection of the end effector and the control system is further configured to: disassociate the first end effector model with the handheld surgical instrument, relinquish control of the alert module based on the pose of the patient boundary and the pose of the modified model of the first end effector, receive the second selection of the end effector, receive the selection of the second security margin, modify a spatial dimension of the second end effector model based on the selected second security margin, determine a pose of the modified second end effector model, determine the pose of the patient boundary associated with the target anatomical feature, and control the handheld surgical instrument based on the pose of the patient boundary and the pose of the modified model of the second end effector.

### BRIEF DESCRIPTION OF THE DRAWINGS

Advantages of the present invention will be readily appreciated as the same becomes better understood by reference to the following detailed description when considered in connection with the accompanying drawings.
FIG. 1 is a perspective view of an exemplary layout of an operating room including a surgical system.
FIG. 2 is a schematic view of an exemplary handheld surgical instrument including a console, a navigation system, and a plurality of interchangeable end effectors.
FIG. 3 is a schematic view of an end effector oriented relative to a target anatomical feature and a set of patient boundaries.
FIG. 4A is an exemplary view of the user interface showing pre-defined options used as initial values for selecting an alert zone settings for a target anatomical feature."
FIG. 4B is an exemplary view of the user interface showing a control bar for controlling one or more alert zones associated with a target anatomical feature.
FIG. 5 is a flowchart describing a method for tracking an end effector of a handheld surgical instrument relative to a target anatomical feature.
FIGS. 6A-6E are exemplary views of a user interface showing different alert zones associated with the target anatomical features of FIG. 4B.
FIG. 7 is a schematic view of an exemplary user interface showing options for selecting an end effector type.
FIG. 8A is a schematic view of an exemplary user interface showing options for selecting a bur size for the end effector.
FIG. 8B is another view of the user interface of FIG. 6A showing options for selecting the bur size for the end effector.
FIG. 9A is a perspective view of an end effector represented by an end effector model oriented relative to a vertebra and boundaries associated with the vertebra.
FIG. 9B is another view of the end effector, vertebra, and boundaries of FIG. 9A, with the end effector represented by a modified end effector model which includes a security margin.
FIG. 9C is another view of the end effector, modified end effector model, vertebra, and boundaries of FIG. 9B in which the modified end effector model is shown crossing at least one of the boundaries.

### DETAILED DESCRIPTION

In the following description, numerous specific details are set forth to provide a thorough understanding of the present invention. It will be apparent, however, to one having ordinary skill in the art that the specific details need not be employed and/or not be employed exactly as described to practice the present invention. In some instances, well-known materials or methods have not been described in detail to avoid obscuring the present invention.

### I. OVERALL SYSTEM

During modern surgical procedures, tracking information may be used to verify whether surgical instruments are following planned surgical pathways and/or maintaining a safe distance away from critical anatomical structures, and correspondingly to provide feedback and/or notify the medical professional executing the procedure when the surgical instrument is at risk of impinging on a critical anatomical structure. To this end, it is also beneficial to provide feedback and/or notify medical professionals when/if a handheld surgical instrument 20 gets close to a critical anatomical structure.

The inventors recognized that some methods of creating models representing surgical instruments and determining collisions between models of surgical instruments and patient structures may not be accurate enough for some surgical procedures and/or feature undesirable latencies. For example, a model created by extending from a center point of an end effector of a surgical instrument may limit that model's sufficient accuracy to application of only spherical end effectors, such as spherical burs. Detecting collisions for models that are created to capture non-spherical end effectors may be very computationally expensive, which may result in undesirable latency. Decreasing the computational expense may involve sacrificing precision, which may not be desirable for a surgical procedure that requires utmost accuracy. As such, there exists a potentially undesirable compromise between flexibility in the types of surgical instruments that may be modeled and collision detection systems that are computationally practical to execute in real time and suitable for handheld surgical instruments.

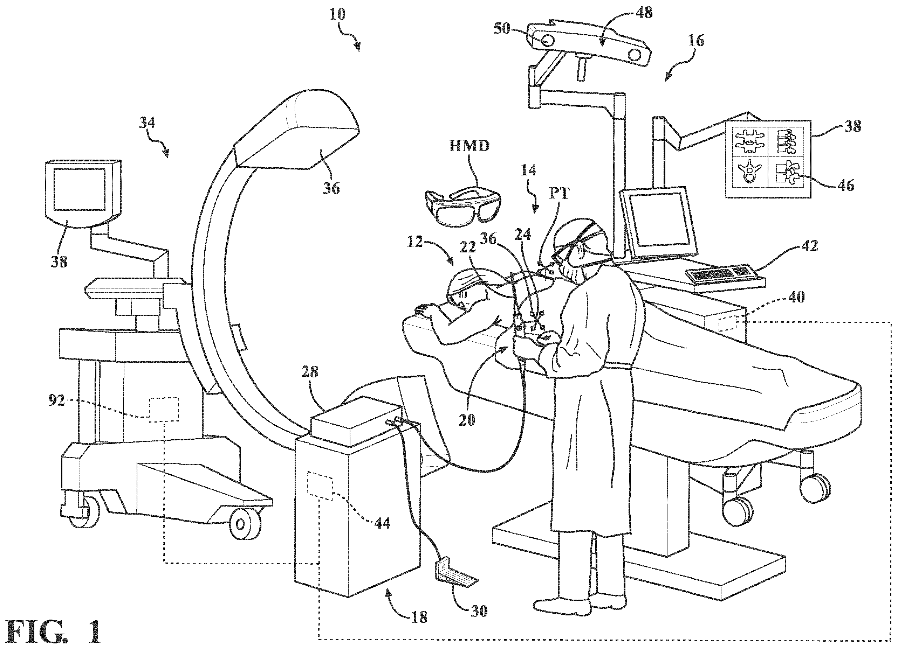

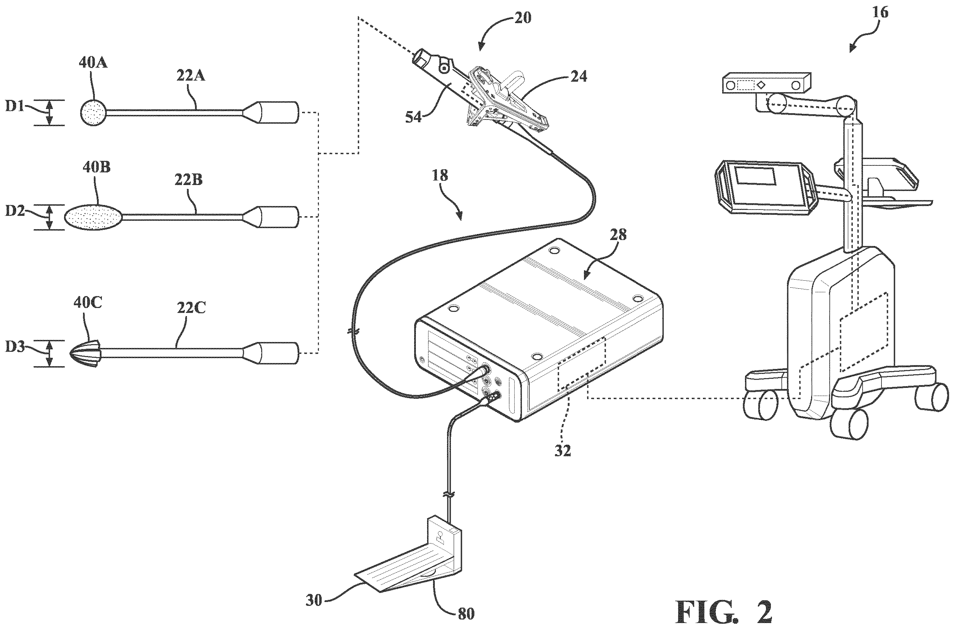

Referring to FIG. 1, an exemplary configuration of a surgical system 10 or surgical suite for performing a medical procedure on a patient 12 is shown. The surgical system 10 may include a surgical navigation system 16 and a surgical instrument assembly 18 (shown in detail in FIG. 2). The surgical navigation system 16 may include a navigation interface that includes one or more display units 38 and one or more user input devices 42. The user input devices 42 may include a touch screen integrated with the display units 38. Additionally or alternatively, the display units 38 of the surgical navigation system 16 may be configured to display one or more graphical user interfaces (GUI) 46, which may include various prompts or data entry boxes for receiving user input. For example, the display unit 38 may be configured to display a text box or prompt that allows the surgeon to manually enter or select the type of surgical procedure to be performed. The display unit 38 may also be configured to display patient data, such as a pre-operative image or scan. The pre-operative image may be based on MRI scans, radiological scans or computed tomography (CT) scans of patient anatomy. The preoperative image may be uploaded to the surgical navigation system 16 and displayed on one of the display units 38. The display units 38 may be further configured to display a surgical plan for a medical procedure overlaid on the patient or image.

The surgical plan may include the surgical pathway for executing the medical procedure, and/or the planned trajectory and/or orientation for the medical instrument during the medical procedure. The surgical plan may also include the position and/or orientation of an implant or medical device to be inserted into a region of interest during the medical procedure, overlayed on the patient data or image. It is contemplated that the surgical navigation system 16 may include the display unit 38 configured to display and/or project an overlaid image of the surgical pathway for executing the medical procedure, and/or the planned trajectory and/or orientation for the medical instrument or implant during the medical procedure. This may include projecting the surgical pathway or implant onto the patient or other surface in the operating room. It may also include a projection of the surgical pathway or planned implant onto a head unit worn by the surgeon, such as a lens, shield, or glasses of the head unit. An exemplary configuration of surgical navigation system 16 including a display unit HMD worn by the surgeon to display a target trajectory and/or target location of a medical procedure is disclosed in International Patent Application No. PCT/IB2018/053130, the entirety of which is hereby incorporated by reference.

The surgical plan may additionally include one or more planned alert zones, which are set around the target anatomical feature to assist in notifying the surgeon that the handheld surgical system is to a defined boundary of interest. The alert zone may be a volume defined using a boundary and a scalar depth offset representing an alert zone margin, which is described in detail in subsequent sections.

The user input device(s) 42 and/or the graphical user interface (GUI) 46 may be configured to allow the surgeon to input or enter patient data and/or modify the surgical plan. The patient data may include patient images, such as pre-operative images of the patient's anatomy. These images may be based on MRI scans, radiological scans or computed tomography (CT) scans of the patient's anatomy. The patient data may also include additional information related to the type of medical procedure being performed, the anatomical features of the patient, the patient's specific medical condition, and/or operating settings for the surgical navigation settings. For example, in performing a spinal surgery, the surgeon may enter information via the user input device(s) 42 and/or the graphical user interface (GUI) 46 related to the specific vertebra on which the medical procedure is being performed. The surgeon may also input various anatomical dimensions related to the vertebrae and/or the size and shape of a medical device or implant to be inserted during the medical procedure. The user input device(s) 42 and/or the graphical user interface (GUI) 46 may also be configured to allow the surgeon to select, edit or manipulate the patient data. For example, the surgeon may identify and/or select anatomical features from the patient data that define regions of interest to the medical procedure. This may include selecting one or more surgical sites 14, such as selecting one or more vertebrae and/or specific areas on the one or more vertebrae, where the medical procedure is to be performed.

The surgeon may also be able to identify critical anatomical structures defining regions of interest to the medical procedure, such as anatomical structures that the surgeon may deem critical. For example, the surgeon may use the user input device(s) 42 and/or the graphical user interface (GUI) 46 to select cortical walls or portions thereof, nerves, blood vessels or similar critical anatomical structures that the surgeon wishes to avoid and establish alert zones surrounding those anatomical structures. The surgeon may also use the user input device(s) 42 and/or the graphical user interface (GUI) 46 to select and/or input a target location, target area to be resected, target trajectory, target depth or similar that is associated with a region of interest to help guide the surgeon in performing the medical procedure.

The system may be configured to utilize segmentation to facilitate identification and spatial positioning of zones and/or boundaries associated with regions of interest to a medical procedure. This segmentation may be performed automatically, semi-automatically, or manually. This segmentation may be done on pre-operative patient data, such as a CT or MRI image, or may be performed on video data, such as endoscopic video data or microscopic video data.

In one example of manual segmentation, the surgeon may utilize the user input device(s) 42 and/or the graphical user interface (GUI) 46 to define a geometric primitive associated with a region of interest to the medical procedure. A method of defining geometric primitives for the purpose of segmentation and visualization of cavities or orifices of the human body may include the steps of: manual pre-segmentation by defining enclosing geometric primitives in a 3D patient image for generating initial envelopes; analyzing the anatomy within pre-segmented geometric primitives; using the result of the analysis for adjustment of the envelopes; and visualizing the envelopes. The adjustment of a visualized envelope can be based on analyzed anatomy using computed voxel affiliations and the adjustment of a visualized cell envelope may be achieved by computing a surface mesh of the voxels which are affiliated completely and/or partially to the cell. Further, the adjustment of a visualized envelope may be achieved by optimizing type, orientation, position and/or size of the enclosing geometric primitive. Exemplary methods and systems for defining a geometric primitive and guiding a surgical instrument are disclosed in U.S. Patent Appl. No. 15/300,414 and U.S. Patent Appl. No. 15/582,637, both of which are hereby incorporated by reference in their entirety.

The user input device(s) 42 and/or the graphical user interface (GUI) 46 may also be configured to enable input of the surgical plan. This may include selecting the surgical instrument to be used and a device and/or implant to be inserted. It may also include identifying a position and/or orientation (i.e., pose) where the device or implant is to be placed within the patient as a region of interest. The user input device(s) 42 and/or the graphical user interface (GUI) 46 may also allow the surgeon to select the parameters of the implant to be inserted, such as the length and/or diameter of a screw to be inserted.

The surgical navigation system 16 may also include a localizer 48. The localizer 48 may be configured to cooperate with the tracking device 24 of the surgical instrument assembly 18 and/or a patient tracker PT to generate tracking data indicative of a pose of the handheld surgical instrument 20 of the surgical instrument assembly 18 and/or of the patient 12, or more particularly one or more regions of interest of the patient 12, in a known coordinate system, such a coordinate system specific to the localizer 48. The patient tracker PT may be coupled to the patient 12 and the localizer 48 may be configured to track the patient tracker PT. To this end, the localizer 48 may include one or more sensors 50. The sensor(s) 50 may include cameras, such as CCD cameras, CMOS cameras, and/or optical image cameras, magnetic sensors, radio frequency sensors, or any other sensor adapted to detect and/or sense the positions and/or orientations (poses) of the one or more tracking devices 24 of the surgical instrument assembly 18 in the known coordinate system. A navigation processor 40 may then be configured to apply a transformation function to such positions and/or orientations based on a known relationship between the tracking device 24 and the handheld surgical instruments 20 in the known coordinate system to determine the positions and/or orientations of the handheld surgical instrument 20 in the known coordinate system. Tracking of the positions and/or orientations of regions of interest of a patient may be similarly performed using one or more patient trackers PT disposed relative to the regions. Description of a suitable localizer 48, and the various technologies that it can utilize, may be found in U.S. Patent Publication No. 2017/0333137, which is hereby incorporated by reference herein in its entirety.

The surgical instrument assembly 18 illustrated in FIG. 1 represents one implementation of the surgical system 10, and that it is contemplated that any number of surgical instrument assemblies 18 may be positioned within the operating room. The surgical instrument assembly 18 may include the handheld surgical instrument 20 and the tracking device 24. Additionally, the handheld surgical instrument 20 may be include a drive motor and/or be configured to receive an end effector 22. The tracking device 24 may include a plurality of markers 26 that are capable of being identified and/or tracked by the surgical navigation system 16. The handheld surgical instrument 20 may be coupled to (i.e., in electrical communication with) a console 28 that is positioned away from the handheld surgical instrument 20. The surgical instrument assembly 18 may also include a foot pedal 30 that is positioned away from the patient 12 and is coupled to the console 28. The tracking device 24 may feature passive reflectors, optical patterns, light emitters, or one or more EM coils.

While not previously discussed, it is also contemplated that the surgical system 10 may further include an imaging system 34, such as CT or MRI imaging device. The imaging system 34 may include a scanner 36 and the display unit 38. The scanner 36 may be utilized to take an image of the surgical site 14 on the patient 12 and display it on the display unit 38. For example, the scanner may include a C-arm configured to be rotated about the patient 12 to produce a plurality of images of the surgical site 14. The imaging system 34 may also include a processor (not shown) including software, as is known by those skilled in the art, which is capable of taking the plurality of images captured by the scanner 36 and producing a 2-D image and/or a 3-D model of the surgical site 14. The display unit 38 may be configured to display the resulting 2-D image and/or 3-D model.

The imaging system 34 may also be in communication with the surgical navigation system 16. For example, the imaging system 34 may be configured to provide pre-operative and/or intraoperative image data, such as the resulting 2-D image and/or 3-D model of the surgical site 14, to the navigation system 16. The system 16 may then be configured to provide the resulting 2-D image and/or 3-D model to the display unit 38, where the surgeon, using the user input device or devices 42 or using algorithms, may identify and/or define the corresponding regions and/or zones around critical anatomical structures. For example, the surgeon may utilize the user input device(s) 42 of the surgical navigation system 16 to define an alert zone around a target anatomical feature, such as a vertebral body, that the surgeon wishes to be made aware of during execution of the medical procedure. The surgeon may utilize the user input device(s) 42 of the surgical navigation system 16 to input and/or modify the planned surgical pathway, patient boundaries, or alert zones to be utilized in executing the medical procedure.

The surgical system 10 may further include a control system 44 in communication with the navigation system 16 and/or the alert module 80. For example, the control system 44 may be in communication with the navigation processor 40 of the navigation system 16, which in turn may be in communication with the GUI 46. Further, the control system may be in communication with an image processor of the imaging system 34 and/or the surgical instrument assembly 18. More specifically, the control system 44 may be in communication with one or more of the handheld surgical instruments 20, the console 28, and/or the instrument processor 32.

The control system 44 may include or be implemented by one or more controllers or one or more processors, each of which may be configured to operate under control of software routines or programs embodied by one or more computer-executable instructions stored in a memory accessible to the controller or processor. The computer-executable instructions may in turn be configured, upon execution by the one or more controllers or processors, to implement the functions, features, processes, and routines of the control system 44 described herein. The control system 44 may be separate from and in communication with other components of the surgical system 10, such as the navigation system 16 and/or the surgical instrument assembly 18. In alternative implementations, the control system 44, or more particularly the functions, features, processes, and routines of the control system 44 described herein, may be implemented by one or more other components of the surgical system 10, such as the navigation processor 40 and/or the instrument processor 32. In other words, the control system 44 may be distributed across multiple devices or systems of the surgical system 10, which may thus be considered as forming the control system 44. Furthermore, the control system 44 may be located on other devices within the operating room, such as a surgical hub, or on a server positioned locally, or on a cloud. Exemplary methods of navigating instruments relative to patient boundaries, alert zones, target trajectories, and/or target depths based on the specific instrument being navigation is described in International Patent Publ. No. 2021/062373, which is incorporated by reference above.

The handheld surgical instrument 20 may also include the tracking device 24 to assist in sensing the position of the handheld surgical instrument 20. As mentioned above, the surgical navigation system 16 may comprise of the localizer 48, including one or more sensors 50. The sensors may comprise cameras, such as CCD cameras, CMOS cameras, and/or optical image cameras, magnetic sensors, radio frequency sensors, or any other sensor adapted to detect and/or sense the position of the tracking device 24 of the handheld surgical instrument 20. One exemplary localizer 48 is capable of detecting radiation or light from the plurality of markers and of generating a localizer signal representative of the detected radiation or light. An exemplary surgical navigation system 16 may be configured to utilize the tracking device 58 with a fixed spatial relation between tracking markers. Description of various suitable localizers that may be utilized can be found in U.S. Patent No. 10,531,926, which is hereby incorporated by reference in its entirety.

### II. INSTRUMENT ASSEMBLY

Referring to FIG. 2, an exemplary configuration of the surgical system 10 is illustrated including the surgical navigation system 16 and the handheld surgical instrument 20. While the handheld surgical instrument 20 illustrated in FIG. 2 is represented as a high-speed bur, it is contemplated that other handheld surgical instruments may be included in the surgical system 10. As shown in FIG. 2, the surgical system 10 may include a plurality of end-effectors 22A, 22B, 22C that are removably couplable to the handpiece 54 of the handheld surgical instrument 20. The end effectors 22A, 22B, 22C may also be referred to as end effectors, surgical attachments, and/or tool attachments. For example, the surgical system 10 may include a first end-effector 22A including a first bur head 40A having a first diameter head D1 and a first shape. The surgical system may also include a second end-effector 22B including a second bur head 40B having a second diameter head D2 and a second shape. The surgical system may also include a third end-effector 22C including a third bur head 40C having a third diameter head D3 with a third shape. The first, second, and third shape being different from one another. It is also contemplated that the head of each the end-effector 40A, 40B, 40C may vary by shape, material, and/or cutting type. It is also contemplated that the length of the shaft may vary from one end-effector 22A, 22B, 22C to the next. The surgical navigation system 16 may be configured to identify which of the end-effectors 22A, 22B, 22C is coupled to the handpiece 54. The navigation system 16 may be configured to identify the end-effectors 22A, 22B, 22C based on a known association with the handpiece 54 and the unique size, shape, and/or arrangement relative to the markers of the instrument tracking devices that is attached to the specific handpiece 54. While burs are provided as one exemplary end effector, other end effectors are also contemplated, such as debriders with various cutting geometries, various saw blades, various twist drill bits, various ultrasonic cutting implements, and the like.

### III. BOUNDARY GENERATION

The surgical navigation system 16 may further include software employed by the navigation processor 40 to control operation of the surgical instrument assembly 18, or more particularly the handheld surgical instrument 20. The software may include a boundary generator. The boundary generator may be implemented on the navigation processor 40, one or more of the instrument processors 32, and/or on other components, such as on the control system 44. An exemplary system for and method of boundary generation may be found in U.S. Patent Publ. No. 2004/0034283A1, which is hereby incorporated by reference herein in its entirety. The boundary generator may also be part of a separate system that operates remotely from the surgical system 10.

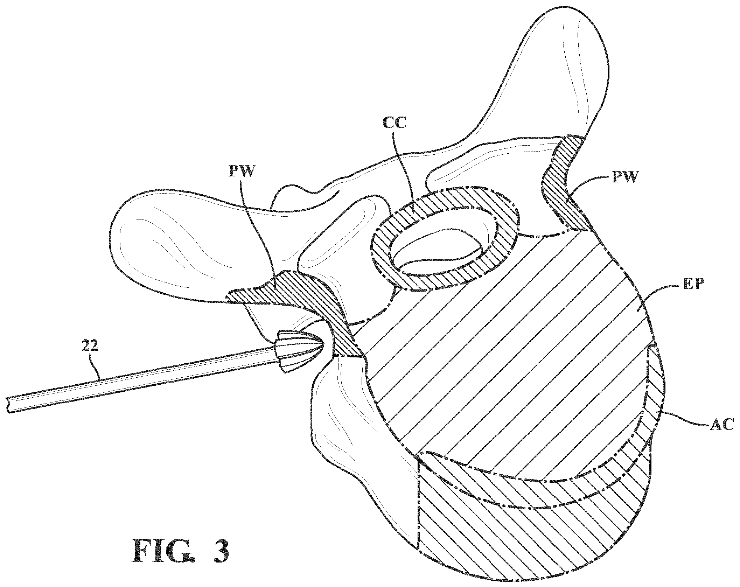

The boundary generator may generate one or more patient boundaries and/or one or more alert zones for constraining operation of the handheld surgical instrument 20. Patient boundaries may be boundaries set on critical patient features that are to be avoided. Alert zones are visualized to be spatially offset from such boundaries. These alert zones, as visualized, define spatial regions in which, an alert module is activated so as to give the surgeon time to receive a notification that they are getting in close proximity to a patient boundary and that they should proceed with caution. Referring to FIG. 3, which shows a schematic view of the end effector 22 oriented relative to a target anatomical feature 60 and a set of alert zones illustrated around the anterior cortex AC, the central canal CC, the end plate EP, and the pedicle wall PW. As mentioned above, the alert zone CC is visualized to be offset from the boundary of the central canal cortical wall so as to give the user time to receive the notification that the handheld surgical instrument 20 is in close proximity to the central canal, which the user would like to avoid. The alert zones may be associated with one or more regions of interest to a medical procedure, including, without limitation, anatomical features or regions targeted for manipulation, resection, or to receive a surgical implant, and/or anatomical features or regions to be avoided.). The alert zones shown in FIG. 3 are an example of possible alert zones for the target anatomical feature 60. The patient boundaries and/or alert zones may also be utilized to control operation of the handheld surgical instrument 20 when the localizer 48 determines that the end effector 22 of the handheld surgical instrument 20 is deemed to be located near the critical structure. For example, the navigation processor 40 may control certain operations/functions of the handheld surgical instrument 20 based on a relationship of the handheld surgical instrument 20 to the boundaries and/or zones (e.g., spatial, velocity, etc.).

Patient boundaries may be one-dimensional (1D), two-dimensional (2D), three-dimensional (3D), and may include a point, line, axis, trajectory, plane (an infinite plane or plane segment bounded by the anatomy or other boundary), volume or other shapes, including complex geometric shapes. Patient boundaries may be represented by pixels, point clouds, voxels, polygon meshes, other 2D or 3D models, combinations thereof, and the like. U.S. Patent Publication No. 2018/0333207 and U.S. Patent No. 8,898,043 are incorporated herein by reference in their entirety, and any of their features may be used to facilitate planning or execution of the surgical procedure. A plurality of boundaries may be used to define the zones likewise associated with the one or more regions of interest.

Patient boundaries may be defined with respect to an anatomical model, such as a 3D bone model. In other words, the points, lines, axes, trajectories, planes, volumes, and the like that are associated with the patient boundaries may be defined in a coordinate system that is fixed relative to a coordinate system of the anatomical model such that tracking of the anatomical model (e.g., via tracking the associated anatomy to which it is registered) also enables tracking of the patient boundary. Further, the anatomical model may be registered to a patient tracker PT (shown in FIG. 1) such that the patient boundaries become associated with the anatomical model and associated coordinate system. The patient boundaries may be implant-specific, e.g., defined based on a size, shape, volume, etc. of an implant and/or patient-specific, e.g., defined based on the patient's anatomy. The patient boundaries may be boundaries that are created pre-operatively, intra-operatively, or combinations thereof. In other words, the patient boundaries may be defined before the surgical procedure begins, during the surgical procedure (including during tissue removal), or combinations thereof. The patient boundaries may be provided in numerous ways, such as by the navigation processor 40 creating them, receiving them from other sources/systems, or the like. The patient boundaries may be stored in memory for retrieval and/or updating.

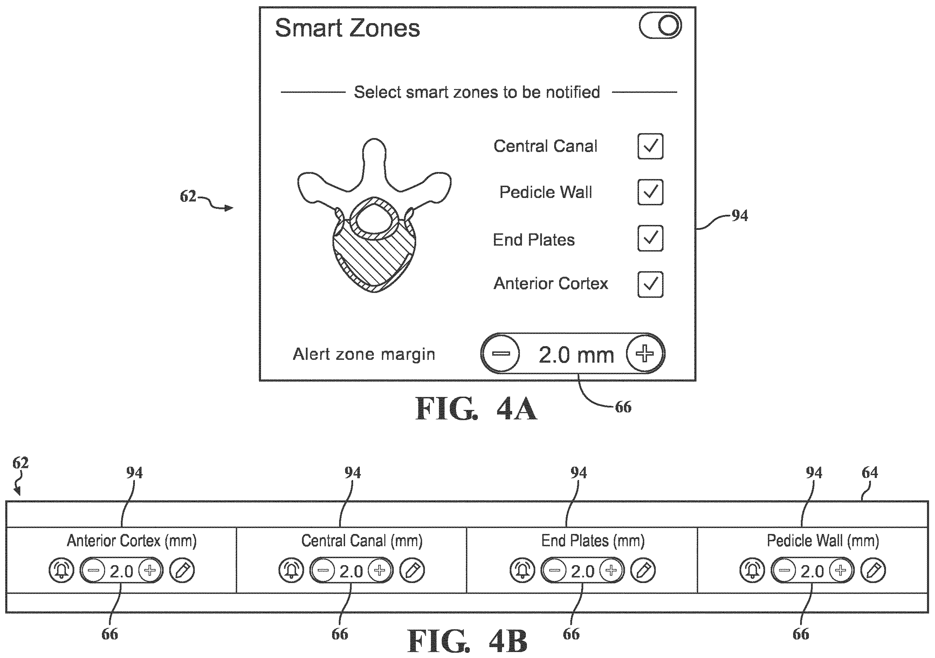

As mentioned above, the patient boundaries and/or alert zones may be selected by the surgeon from a populated list of patient boundaries provided by the boundary generator software of the surgical navigation system 16. An example of the alert zone selection can be seen in FIGS. 4A and 4B. FIG. 4A shows an exemplary view of the user interface 62 showing pre-defined options used as initial values for selecting alert zone settings for the target anatomical feature 60. The alert zone settings may include selecting the different alert zones and/or may also include selecting the alert zone margin 66. The alert zone margin 66 may be applied across all selected alert zones (depicted with a check mark), or may be separately selected for each zone. FIG. 4B shows an exemplary view of a user interface 62 showing a control bar 64 that is populated with different alert zones for the target anatomical feature 60. The different regions shown in the control bar 64 include an anterior cortex, central canal, end plates, and pedicle wall but may be populated with other regions depending on the target anatomical feature 60 and/or the medical procedure being performed. Each of these regions can have a different alert zone margin 66 typically measured in millimeters and may be either individually selected using an input mechanism 94 shown in FIG. 4B or selected using the input mechanism 94 in FIG. 4A wherein the alert zone margin 66 may be specified for all selected alert zones. The surgeon can customize the alert zone margin 66 for each alert zone depending on the medical procedure and surgical instrumentation being used. As an example, the surgeon may create the alert zone margin 66 to be higher for one alert zone, which potentially provide earlier notifications to the surgeon to proceed with caution as the handheld surgical instrument 20 gets closer in proximity the corresponding critical part of the target anatomical feature. The surgeon may also create the alert zone margin 66 to be a lower value, which allows the handheld surgical instrument 20 to get closer in proximity to a critical part of the target anatomical feature before receiving such notifications.

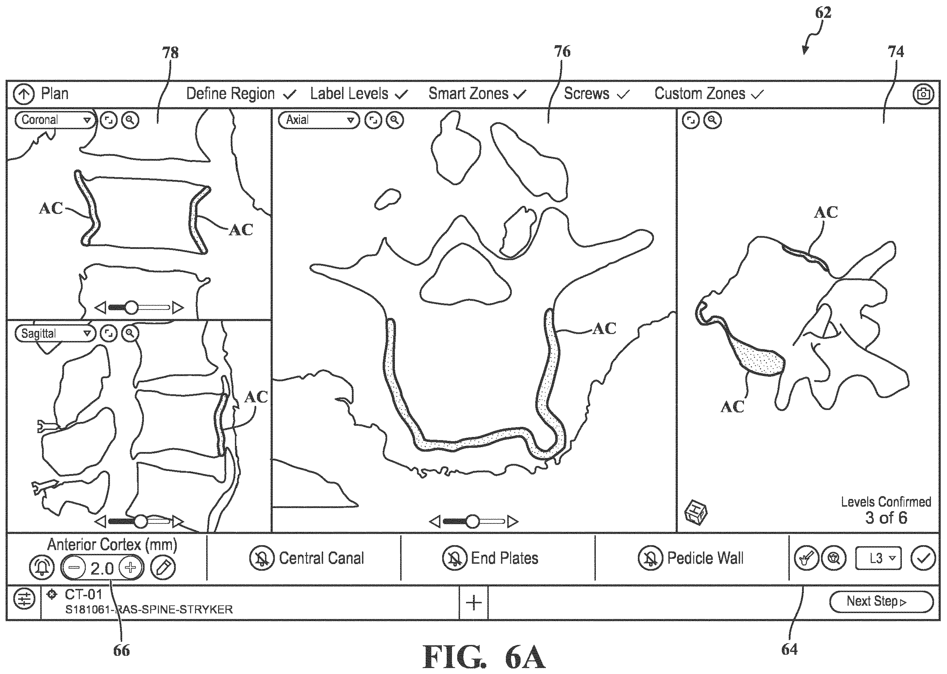

Once the alert zone margins 66 for the alert zones are manually selected or picked from a populated list of patient boundaries provided by the boundary generator software of the surgical navigation system 16, preoperative medical images may be generated. Examples of such preoperative medical images are shown in FIGS. 6A-6E. Each preoperative medical image may include a 3-dimensional view 74 of the target anatomical feature 60, a 2-dimensional view 76 of the target anatomical feature 60, and/or a macro view 78, which are all shown in the user interface 62. The macro view 78 may show a high-level representation of the anatomical structure in which the target anatomical feature 60 is located. The different views 74, 76, 78 provide the surgeon with information regarding the location of the selected patient boundaries and the alert zones.

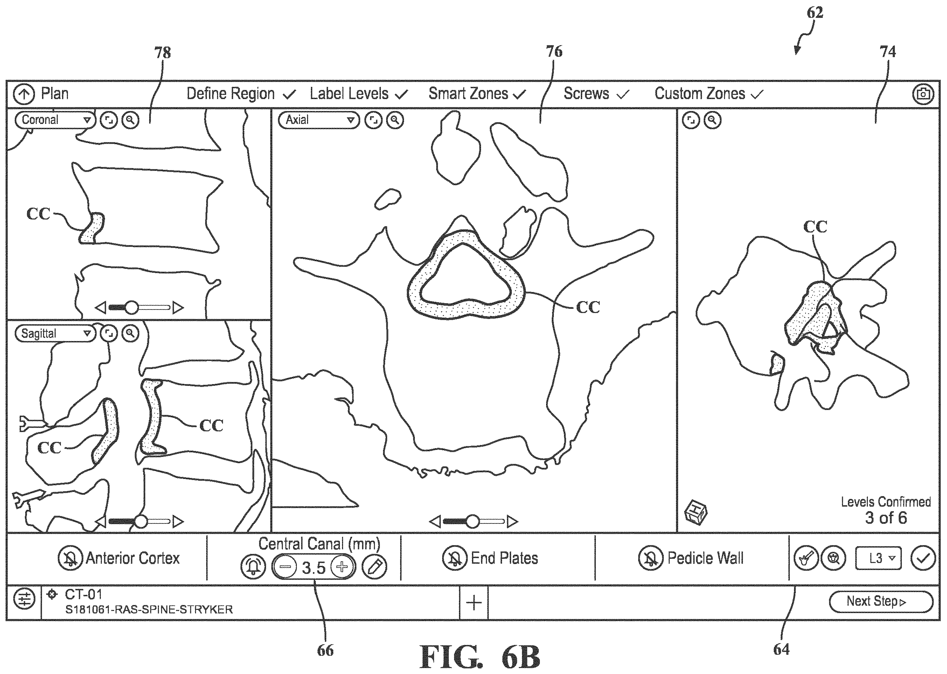

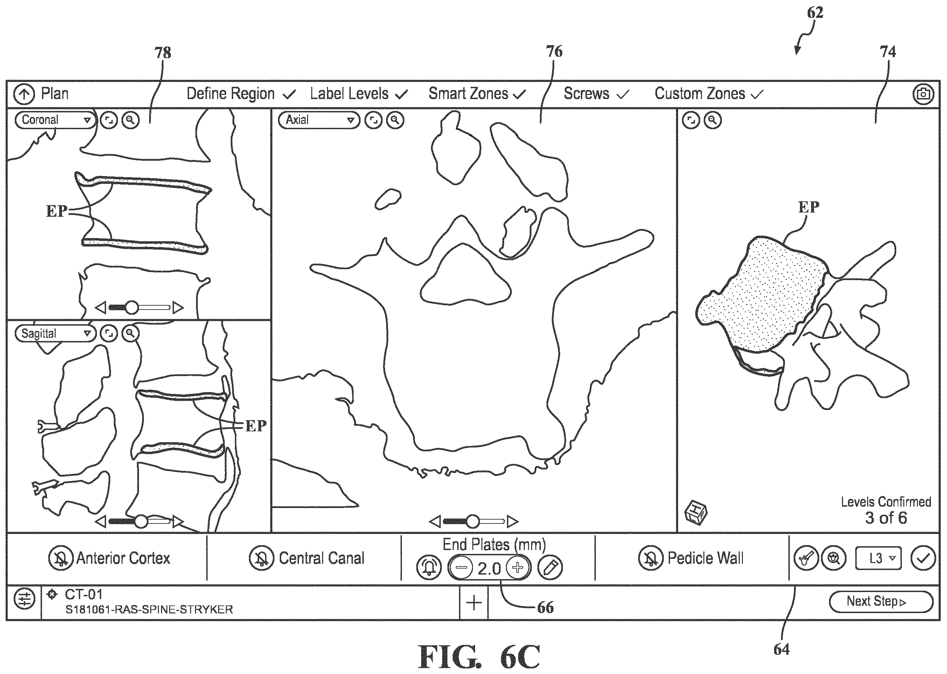

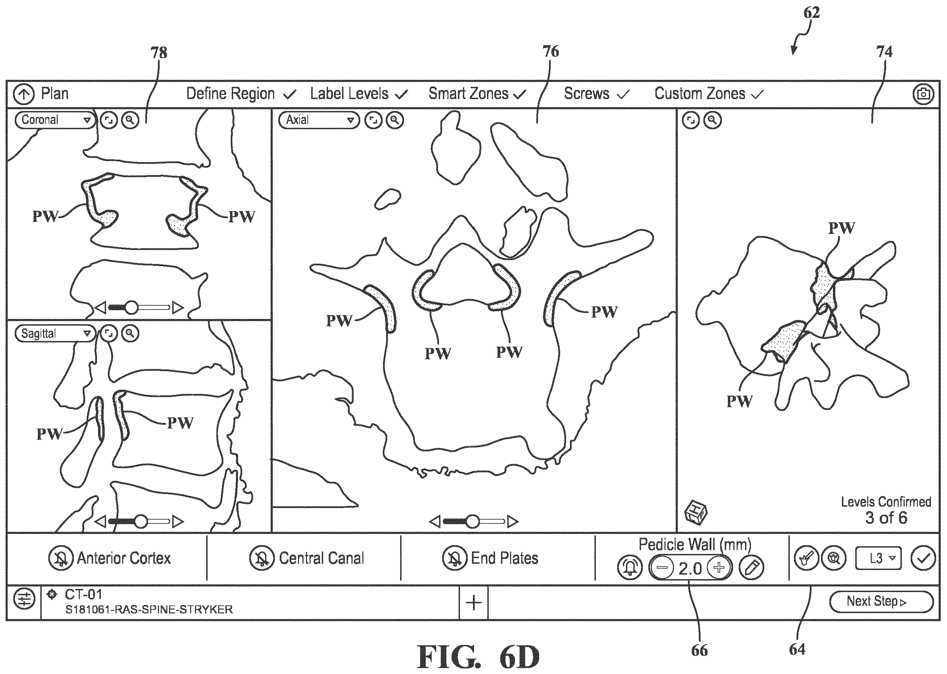

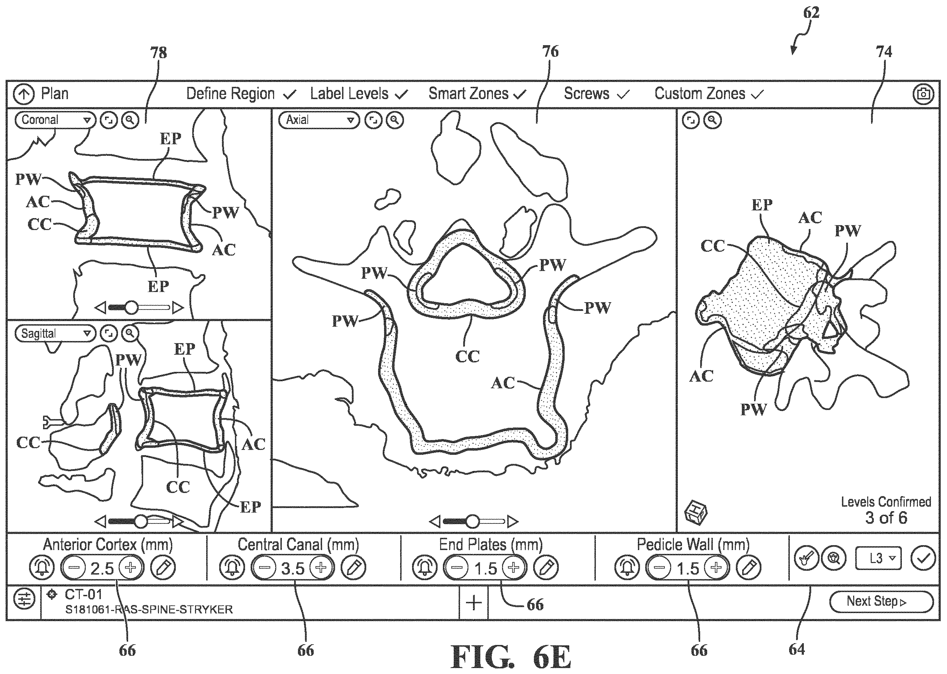

Although the preoperative medical images in FIGS. 6A-6E show similar views 74, 76, 78, they each include different patient boundaries and alert zones with associated alert zone margins 66 shown in each respective control bar 64. For example, FIG. 6A shows the alert zone AC applied to the anterior cortex with the alert zone margin 66 set at 2.0 mm. This means that the surgeon would like to be notified when the end effector 22 of the handheld surgical instrument 20 is within 2.0 mm of the anterior cortex. However, in FIG. 6B, the preoperative medical image 68 also shows the alert zone CC applied to the central canal but with the alert zone margin 66 of 3.5 mm, which will provide the user with notifications when the end effector 22 of the handheld surgical instrument 20 is within 3.5 mm of the central canal. Another example is shown in FIG. 6C where the preoperative image shows the alert zone EP applied to the end plate with the alert zone margin 66 of 2.0 mm, which will provide the user with notifications when the end effector 22 of the handheld surgical instrument 20 is within 2.0 mm of the end plate. In yet another example, FIG. 6D shows the preoperative image with the alert zone PW applied to the pedicle wall with the alert zone margin 66 of 2.0 mm, which will provide the user with notifications when the end effector 22 of the handheld surgical instrument 20 is within 2.0 mm of the pedicle wall. Finally, FIG. 6E shows the preoperative image with four alert zones (AC, CC, EP, PW) applied to the anterior cortex, central canal, end plates, and the pedicle wall with varying alert zone margins 66. This will allow the user to receive notifications based upon all selected alert zones, which in the example shown in FIG. 6E would include the anterior cortex, central canal, end plate, and pedicle wall. In other words, the surgeon can select the alert zones that are needed for the specific medical procedure and visualize them and be notified of proximity individually (as shown in FIGS. 6A-6D) or all together (shown in FIG. 6E) to help ensure a successful medical procedure.

### IV. END EFFECTOR SELECTION AND MODELING

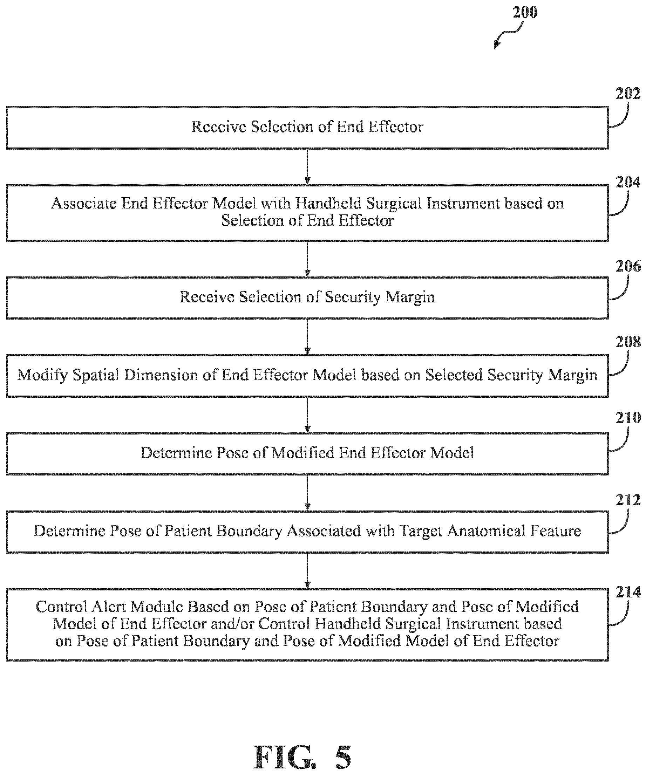

As mentioned above, the surgical navigation system 16 may be configured to track the handheld surgical instrument 20 (e.g., the end effector 22) relative to target anatomical features. Referring to FIG. 5, a flowchart describing a method 200 for tracking the handheld surgical instrument relative to the target anatomical feature is shown.

As shown in FIG. 5, the first step 202 of the method 200 is to receive the selection of the end effector 22 and the second step 204 is to associate an end effector model 82 with the handheld surgical instrument 20 based on the selection of the end effector 22. This association may be based on a stored identity of the handheld surgical instrument 20 or attached tracker, or a stored correspondence between a tracker geometry and the handheld surgical instrument 20.

The selection of the end effector 22 may be chosen for a given surgical procedure based on attributes and characteristics of the end effector 22. These attributes and characteristics may include precision, dexterity, safety, and/or procedure-specific functionality of the end effector 22. For example, end effectors 22 tailored to their unique requirements of the planned resection may be selected for the medical procedure.

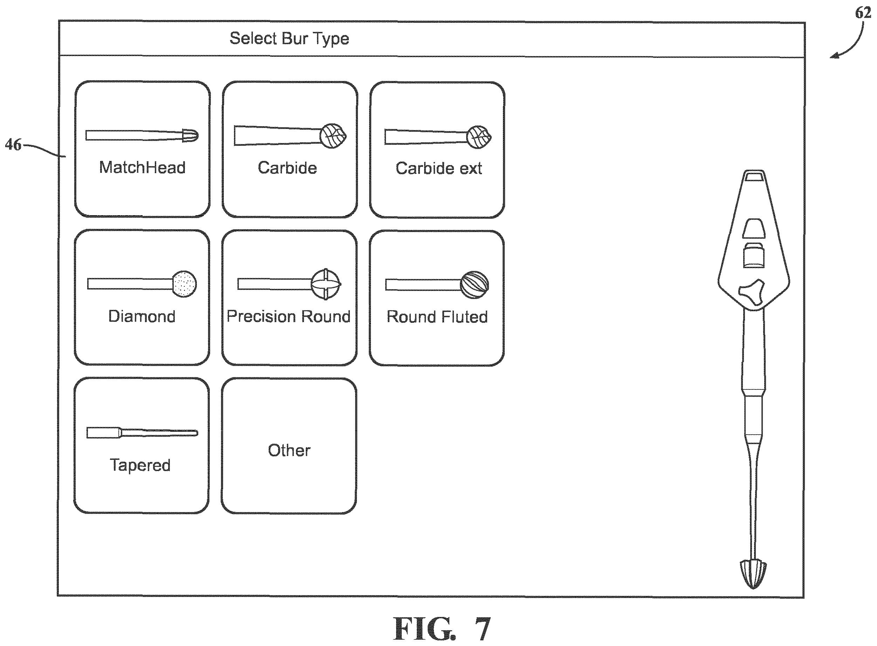

Referring to FIG. 7, which shows an exemplary view of the user interface 62 showing the GUI 46 for selecting the end effector 22. FIG. 7 shows how the user may select the end effector 22 by specifically selecting the bur type, as an example. The bur types and/or shapes shown include matchhead, carbide, carbide extended, diamond, precision round, round fluted, tapered, and other. The different bur types shown in FIG. 7 may have different shapes, which affect how the end effector is ultimately modeled. For example, the model for the matchhead end effector would require its model to be constructed using differently shaped meshes than a model created for a diamond end effector. It should be noted that the GUI 46 may show different end effectors 22 altogether depending on the specific medical procedure to be performed.

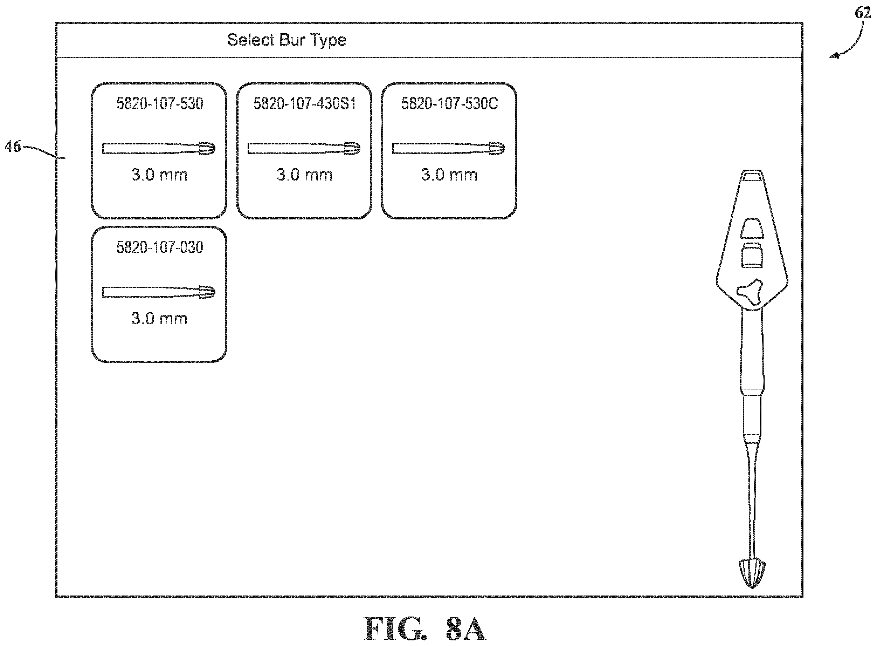

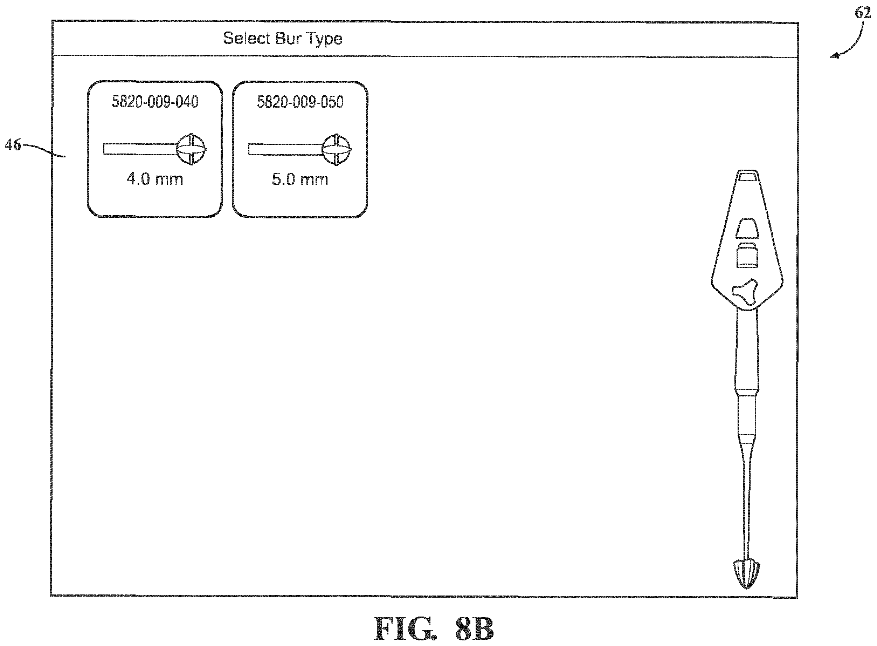

In addition to choosing the end effector 22 type, the user may also choose the specific size of the end effector 22, an example of which is shown in FIGS. 8A and 8B. FIG. 8A shows an exemplary view of the user interface 62 showing options for selecting a bur size for the end effector 22. FIG. 8B is another exemplary view of the user interface 62 showing options for selecting the bur size for the end effector 22. As an example, FIG. 8A shows options for choosing between different bur sizes measured at 3.0 mm, whereas FIG. 8B shows options for choosing between different bur sizes measured between 4.0 and 5.0 mm. The size of the end effector 22 may affect how the modeling is done. For example, the model for a matchhead end effector with a size of 3.0 mm may be different than the model for a matchhead end effector with a size of 3.5 mm. The end effector 22 is chosen for the specific medical procedure, and is subsequently modeled with the end effector model 82 by the control system, which is discussed in detail below.

### V. END EFFECTOR MODEL

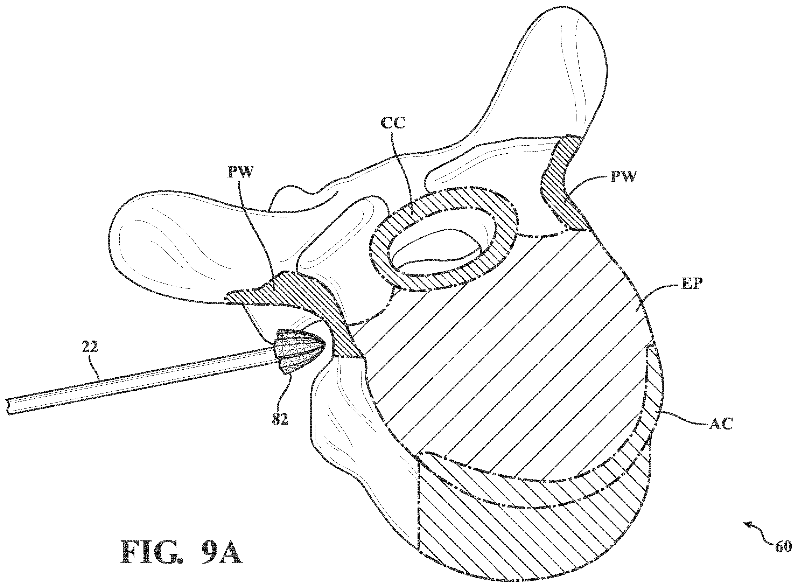

Referring back to FIGS. 5 and 9A, the second step 204 of the method 200 is to associate the end effector model 82 with the handheld surgical instrument 20 based on the selection of the end effector 22. The association between the end effector model 82 and the handheld surgical instrument 20 is done to assist in tracking the location of the end effector 22 throughout the surgical procedure. FIG. 9A shows the end effector 22 as being represented by the end effector model 82 oriented in an exemplary position relative to the patient's target anatomical feature 60 and the defined set of patient boundaries (AC, CC, EP, and PW) corresponding to the anterior cortex, central canal, end plate, and pedicle wall, respectively.

The end effector model 82 may be represented as a polygon mesh. A polygon mesh may be a collection of vertices, edges, and faces that define the shape of an object such as the end effector 22. Vertices represent positions in 3-dimensional space wherein each vertex may include additional information such as color, normal vectors, and texture coordinates. Edges connect pairs of vertices and define the relationship between adjacent points. Faces are closed sets of edges, which are most commonly represented as triangles forming a triangle mesh. However, the faces may also be more complex including concave polygons as well as polygons containing holes. Modeling and tracking such a model can be computationally expensive to do with a high level of precision.

Further, the end effector model 82 may include a resolution, which may be optionally selected by the control system 44 based on a current stage of a surgical procedure, a type of end effector 22, and/or the pose of the patient boundaries associated with the target anatomical feature 60. The resolution may impact the number of vertices that represent the end effector model 82. Typically, the higher the resolution, the higher the precision which may be necessary for certain surgical procedures and/or to accommodate the complexity of the end effector 22 shape and size. However, as the resolution is increased to accommodate the need for higher precision, the computational expense of modeling and tracking such a model may also increase.

### VI. SECURITY MARGIN

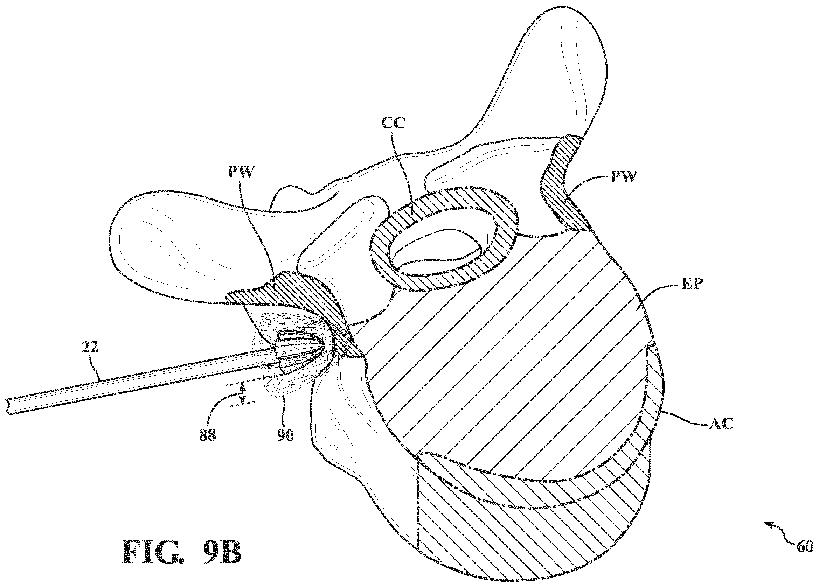

Referring to FIGS. 4 and 9B, a third step 206 of the method 200 is to receive the selection of a security margin 88. It should be noted that the security margin 88 functions in the same way as the alert zone margin 66 regarding how the control system 44 notifies the user when a collision occurs, but the security margin 88 and the alert zone margin 66 each have different illustrative purposes described herein. The security margin 88 is the distance the end effector model 82 is extended away from the physical end effector 22 thereby creating a modified end effector model 90, which can be seen in FIG. 9B. The security margin 88 is not typically visualized for the surgeon but is shown in FIG. 9B for illustrative purposes. Whereas the alert zone margin 66 (such as the ones shown in FIGS. 6A-6E) are typically visualized so that the user can understand where the alert zones are located relative to anatomical features of the patient 12. However, despite the security margin 88 and the alert zone margin 66 illustrating different elements, the control system 44 treats them as the same in regard to how the control system 44 notifies the user when a collision occurs. In other words, when the user manually selects the alert zone margin 66, the control system 44 uses that value to select the security margin 88 to create the modified end effector model 90. For example, if the user selects the alert zone margin 66 to be 2.0 mm, that means that the user would like to be notified that a collision has occurred when the distance between the corresponding patient boundary and the end effector 22 is 2.0 mm.

The security margin 88 may be chosen manually via a user input. As mentioned above, the user may select the different alert zones that are needed and manually specify the alert zone margin 66 for all alert zones (as shown in FIG. 4A) or specify different alert zone margins for each alert zone (as shown in FIG. 4B). The control system 44 then creates the modified end effector model 90 taking into account the manually inputted alert zone margin 66.

Additionally or alternatively, the security margin 88 may be chosen based on the selection of the end effector 22. For example, the user interface 62 shown in FIG. 7 shows different kinds of end effectors 22 that each may have a different recommended associated security margin 88 depending on the size and shape of end effector 22. For example, a matchhead end effector may have a different associated security margin 88 than a tapered end effector. It is also possible for two different end effector 22 types to have the same security margin 88.

The security margin 88 may also be changed intraoperatively via the user input. For example, the security margin 88 may also be changed to accommodate a complication during surgery, in which case the surgeon may change the security margin 88 to be larger or smaller depending on the required surgical step. For example, during the course of a surgical procedure, the surgeon may want to avoid a region within the surgical site 14 and may increase the security margin 88 to a larger value as the end effector 22 gets closer in proximity to the region that is to be avoided. The surgeon may also change end effectors 88 intraoperatively, which may change the required security margin 88, which is discussed in detail further below. Additionally or alternatively, the user interface 62 shown in FIGS. 8A and 8B show the selection of bur size for the end effector 22, which may also impact the selection of the security margin 88. For example, a diamond end effector with a size of 3.0 mm may have a different security margin 88 than a diamond end effector with a size of 4.0 mm. In other words, the selection of the end effector 22 in both type, size, shape or other attributes may change the selection of the security margin 88 that is received by the control system to accommodate a given surgical procedure.

The security margin 88 may be chosen based on the type of surgical procedure. Some surgical procedures may require more precision than others which may impact the selection of the security margin 88. For example, surgical procedures that may require operating near a critical anatomical structure such as the spinal cord typically require a high level of precision. As can be seen in FIG. 6B, the alert zone margin 66 may be set to a value of 3.5 mm around the alert zone CC corresponding to the central canal. The value may be set to a higher value to provide the user with notifications at a greater distance from the critical anatomical structure. Alternatively, there are some surgical procedures that may require less precision than the procedures noted above. Such procedures may include hernia repair, tonsillectomies, or appendectomies. For example, the alert zone margin 66 around the alert zone AC corresponding to the anterior cortex shown in FIG. 6A may have a smaller value allowing the user to get closer to that anatomical structure prior to receiving warnings from the control system 44 of a collision. In other words, the type of surgical procedure may impact the selection of the security margin 88 to help ensure a successful procedure depending on the potential requirements associated with that procedure. For example, surgical procedures such as a total knee replacement surgery may likely require a different security margin than a spinal laminectomy surgery. Although a total knee replacement surgery requires precision in removing portions of bone to make room for a surgical implant, the surgeon is likely working with more room with the surgical site than a spinal laminectomy surgery. Spinal surgery such as a laminectomy may likely have less room within the surgical site for the surgeon to operate, which may require a smaller security margin 88. In addition to how much room the surgeon has in the surgical site, the patient anatomical feature that is to be operated on may dictate the value of the security margin 88. For example, surgical procedures such as spinal decompression may include removing bony walls of the vertebrae and any bone spurs to open up the spinal column to alleviate pressure on the nerves, which may require the surgeon to get in close proximity to the spinal cord. In such surgical procedures, the security margin may be set to a value that allows the surgeon to properly avoid the spinal cord.

The security margin 88 may also be selected based on a current stage of a surgical procedure and/or the pose of the patient boundaries associated with the target anatomical feature 60. A given medical procedure may include operating on multiple patient structures, which may require the security margin 88 be set to a first value for one portion of the surgical procedure and then set to a second value for the second portion of the surgical procedure. For example, as seen in FIG. 6E, the user could designate multiple alert zone margins with different values. In FIG. 6E, the alert zone margin 66 for the alert zone AC pertaining to the anterior cortex is set to a value of 2.5 mm, whereas the alert zone margin 66 for the alert zone CC pertaining to the central canal is set to a value of 3.5 mm.

Referring to FIG. 9B, which shows the modified end effector model 90 and the security margin 88. The control system 44 modifies a spatial dimension of the end effector model 82 based on the selected security margin 88 to create the modified end effector model 90. As described above, the selected security margin 88 may be determined based on the alert zone margin 66, which may be manually input by the user. FIG. 9B shows an example of the modified end effector model 90 wherein the end effector model 82 is extended from the end effector 22 by the security margin 88. Essentially, the modified end effector model 90 shown in FIG. 9B is created by increasing the size of the end effector model 82 by the security margin 88 in three spatial dimensions. However, it is contemplated that the end effector model 82 may be created by extending the end effector model 82 by one and/or two spatial dimensions. For example, if the end effector 22 was a drill bit, the modified end effector model 90 may be extended by the security margin 88 in only one spatial dimension along the drill bit's axis. The end effector model 82 may also be a two-dimensional model and the security margin 88 may be applied to one or both of the two-dimensions. For example, if the end effector 22 is a drill bit, the security margin 88 may be applied to the length of the drill bit (as mentioned above) and may also be applied to the radius of the drill bit thereby extending the end effector model 82 in two spatial dimensions.

### VII. NOTIFICATIONS BASED ON COLLISION

Still referring to FIG. 5, the next step 210 is to determine a pose of the modified end effector model 90, which includes tracking the modified end effector model 90. However, as previously mentioned, tracking each and every vertex of the modified end effector model 90 can be computationally expensive for the controls system 44. This computational expense may introduce latency in tracking the end effector 22 of the handheld surgical instrument 20, which may lead to negatively impact the success of the medical procedure. One way to lessen that computational expense is instead of tracking every vertex of the modified end effector model 90 in relation to the patient boundaries, the control system 44 tracks a collision between the nearest vertex of the modified end effector model 90 and the patient boundaries. As the user moves the handheld surgical instrument 20, the nearest vertex of the modified end effector model 90 may change from one vertex to another. This means that the control system 44 does not need to track every vertex individually, which lessens the computational expense. This approach lessens the computational expense while maintaining high precision during a surgical procedure. In response to such a collision, the control system 44 provides notifications to the user, such as those discussed in detail below.

The next step 214 as shown in FIG. 5 is for the control system 44 to be further configured to control an alert module 80 based on the pose of the patient boundary and the pose of the modified end effector model 90 of the end effector 22. The alert module 80 may be selected from a group including a display device (such as the display unit 38 and/or head-mounted display HMD shown in FIG. 1), a haptic feedback device (such as a vibration motor onboard the foot pedal 30), a speaker onboard the navigation system and/or a head-mounted display HMD. Other devices capable of delivering notifications to the user are also contemplated.

In response to a collision between the modified end effector model 90 and the patient boundary, the alert module 80 may provide notifications to the user. For example, if the alert module 80 is the display unit 38, the alert module 80 may control the display unit 38 to display a notification such as a visual warning or a flashing screen. As another example, if the alert module 80 is the haptic feedback device, the alert module 80 may provide a tactile feedback to the user such that the user will feel the tactile feedback and be alerted to the collision. As an additional example, if the alert module 80 is the speaker, the alert module 80 may output an audible alert to the user in response to the collision. In other words, the alert module 80 provides notifications to the user in some way to alert the user that a collision has occurred and the user can take subsequent actions to help avoid any patient injury or complications to the surgical procedure.

Additionally or alternatively, the control system 44 may be configured to control the handheld surgical instrument 20 based on the pose of the patient boundary 72 and the pose of the modified end effector model 90 of the end effector 22. For example, in response to a collision, the control system 44 may control the handheld surgical instrument 20 by reducing an operating speed of the drive motor including reducing the operating speed to a value of zero. For example, if a collision occurs, the speed of the drive motor of the end effector 22 will be reduced from the normal operating speed to a lower speed that is sensed by the user and corrective actions can be implemented. The control system 44 may also control the handheld surgical instrument 20 by applying engine braking or changing the voltage and/or current supplied to the drive motor. For example, the control system 44 may lower the current supplied to the handheld surgical instrument 20 which may constrain movement or prevent some functionality thereby notifying the surgeon that a collision between the modified end effector model 90 and the patient boundary has occurred. Additionally or alternatively, the control system 44 may control the handheld surgical instrument 20 by controlling the console 28 thereby limiting or changing functionality in the handheld surgical instrument 20. Furthermore, in the case that the handheld surgical instrument 20 is an electrosurgical instrument, the control system 44 may control an operating parameter of the instrument based on the boundary and the modified end effector model 90.

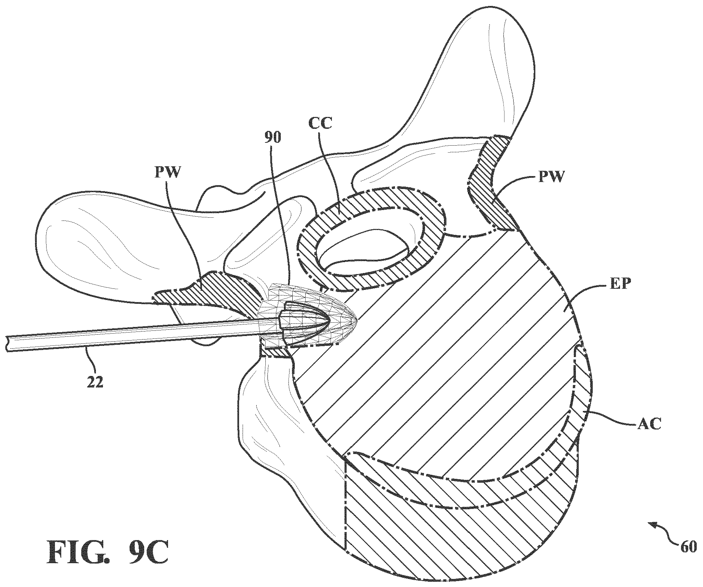

An illustration of an exemplary collision is shown in FIG. 9C. The collision between the modified end effector model 90 and the patient boundary is shown by illustrating part of the modified end effector model 90 colliding with the alert zone EP corresponding to the patient boundary on the end plate of the vertebral body. As mentioned above, the security margin 88 is selected to provide some distance between the target anatomical feature 60 and the end effector 22 itself. As shown in FIG. 9C, although the modified end effector model 90 is colliding with the alert zone EP, the end effector 22 itself is not coming into direct contact with the end plate of the vertebral body. However, the collision of the modified end effector model 90 and the alert zone EP allows the control system 44 to control the alert module 80 to provide notifications to the user that a collision has occurred prior to the end effector 22 itself coming into contact with the end plate. As previously mentioned, such notifications may include controlling the alert module 80 and/or may include controlling the handheld surgical instrument 20. In other words, the control system 44 by controlling the alert module 80 and/or the handheld surgical instrument 20 may alert the user if there is a collision between the modified end effector model 90 and the patient boundary to alert the user that such a collision has occurred and allows time for the user to implement corrective measures.

Some surgical procedures may require multiple end effectors 22. The control system is further configured to accommodate the user switching end effectors 22 as is required for the specific surgical procedure. As an example, a surgical procedure may require two end effectors 22 (a first end effector and a second effector). When the user is done using the first end effector, the control system 44 disassociates the first end effector model with the handheld surgical instrument 20. The control system 44 then relinquishes control of the alert module 80 (and/or the handheld surgical instrument 20) regarding the first end effector, which is based on the pose of the patient boundary and the pose of the modified model of the first end effector. The control system 44 then receives the second selection of the end effector 22 and the selection of the second security margin 88, and modifies a spatial dimension of the second end effector model 82 based on the selected second security margin 88. The control system 44 then determines a pose of the modified second end effector model and the pose of the patient boundary associated with the target anatomical feature 60. The control system 44 then controls the alert module 80 and/or the handheld surgical instrument 20 based on the pose of the patient boundary and the pose of the modified model of the second end effector. In other words, the control system 44 is able to accommodate users who need to switch end effectors 22 during a surgical procedure seamlessly.

Several implementations have been discussed in the foregoing description. However, the implementations discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A surgical navigation system (16) comprising:
a handheld surgical instrument (20) configured to receive an end effector (22);
a localizer (18) configured to determine a pose of the handheld surgical instrument (20),
an alert module (80) for providing notifications to a user; and
a control system (44) in communication with the localizer (18) and the alert module (80), the control system (44) configured to:
receive a selection of the end effector (22),
associate an end effector model (82) with the handheld surgical instrument (20) based on the selection of the end effector (22),
receive a selection of a security margin (88),
modify a spatial dimension of the end effector model (82) based on the selected security margin (88),
determine a pose of the modified end effector model (90),
determine a pose of a patient boundary (72) associated with a target anatomical feature (60), and
control the alert module (80) based on the pose of the patient boundary (72) and the pose of the modified model of the end effector (90).

2. The surgical navigation system (16) of claim 1, wherein the surgical navigation system (16) further includes a console (28) in electrical communication with the handheld surgical instrument (20).

3. The surgical navigation system (16) of claim 1 or 2, wherein:
the surgical navigation system (16) includes a patient tracker (PT) coupled to a patient;
the localizer (18) is configured to track the patient tracker (PT); and
the control system (44) is configured to associate the patient boundary (72) of the target anatomical feature (60) with the patient by associating the pose of the patient boundary (72) with a pose of the patient tracker (PT).

4. The surgical navigation system (16) of any of claims 1 to 3, wherein the control system (44) is configured to receive the selection of the security margin (88) based on one or more of the selection of the end effector (22) and a type of surgical procedure.

5. The surgical navigation system (16) of any of claims 1 to 4, wherein the security margin (88) is based on at least one of the following:
a current stage of a surgical procedure;
the pose of the patient boundary (72) associated with the target anatomical feature (60); and/or
a user input.

6. The surgical navigation system (16) of claim 5, wherein the security margin (88) is based on the user input and the user input includes a type of end effector (22).

7. The surgical navigation system (16) of any of claims 1 to 6, wherein one or more of the end effector model (82) and the patient boundary (72) is a polygon mesh.

8. The surgical navigation system (16) of any of claims 1 to 7, wherein the end effector model (82) includes a resolution and the control system (44) is configured to select the resolution based on:
a current stage of a surgical procedure;
a type of end effector (22); and/or
the pose of the patient boundary (72) associated with the target anatomical feature (60).

9. The surgical navigation system (16) of any of claims 1 to 8, wherein the control system (44) is further configured to control the alert module (80) based on a collision between the modified end effector model (90) and the patient boundary (72).

10. The surgical navigation system (16) of any of claims 1 to 9, wherein the alert module (80) is selected from a group including:
a display device;
a haptic feedback device; and/or
a speaker.

11. The surgical navigation system (16) of claim 10, wherein the alert module (80) is the display device and the control system (44) is further configured to control the display device to display a notification in response to a collision between the modified end effector model (90) and the patient boundary (72).

12. The surgical navigation system (16) of claim 10, wherein the alert module (80) is the haptic feedback device and the control system (44) is further configured to control the haptic feedback device to provide a tactile feedback in response to a collision between the modified end effector model (90) and the patient boundary (72).

13. The surgical navigation system (16) of claim 10, wherein the alert module (80) is the speaker and the control system (44) is further configured to control the speaker to output an audible alert in response to a collision between the modified end effector model (90) and the patient boundary (72).

14. The surgical navigation system (16) of any of claims 1 to 13, wherein the selection of the end effector (22) is realized as a first selection of the end effector (22) and a second selection of the end effector (22) and the control system (44) is further configured to:
disassociate the first end effector model (82) with the handheld surgical instrument (20),
relinquish control of the alert module (80) based on the pose of the patient boundary (72) and the pose of the modified model of the first end effector (90),
receive the second selection of the end effector (22),
receive the second selection of the security margin (88),
modify a spatial dimension of the second end effector model (82) based on the selected second security margin (88),
determine a pose of the modified second end effector model (90),
determine the pose of the patient boundary (72) associated with the target anatomical feature (60), and
control the alert module (80) based on the pose of the patient boundary (72) and the pose of the modified model of the second end effector (90).

15. The surgical navigation system (16) of any of claims 1 to 14, wherein the end effector (22) includes a non-spherical bur.
